# EUROPEAN PATENT SPECIFICATION

(11) **EP 1 006 934 B1**
(45) Date of publication and mention of the grant of the patent: **29.09.2004**
(21) Application number: 98933017.0
(22) Date of filing: 01.07.1998
(51) Int. Cl.: A61F 2/08

(54) **APPARATUS FOR ANCHORING AUTOLOGOUS OR ARTIFICIAL TENDON GRAFTS IN BONE**
VORRICHTUNG ZUM VERANKERN VON KÖRPEREIGENEN ODER KÜNSTLICHEN SEHNENTRANSPLANTATEN IN KNOCHEN
APPAREIL D'ANCRAGE DE GREFFES DE TENDONS AUTOLOGUES OU ARTIFICIELS DANS DE L'OS

(30) Priority: 03.07.1997 US 887580
(43) Date of publication of application: 14.06.2000
(73) Proprietor: Ethicon, Inc., Somerville, NJ 08876 (US)
(72) Inventor: HART, Rickey, D., Plainville, MA 02762 (US)
(74) Representative: Greenwood, John David
(86) International application number: PCT/US1998/013636
(87) International publication number: WO 1999/001084

(56) References cited:
- WO-A-95/15726
- WO-A-98/22048
- FR-A- 2 728 779
- US-A- 4 828 562
- US-A- 4 870 957

## Description

This invention pertains to surgical apparatus for attaching autologous or artificial tendon grafts to bone as the one disclosed in document US-A-4 870 957. The invention has applications in, for example, repair of the anterior cruciate ligament (ACL) of the knee. It may also be used, for example, for repair of other ligaments such as of the elbow or ankle.

It is not uncommon for ligaments and other soft tissue to tear or detach from bone. Athletes, for example, often suffer tears or other injuries to the anterior cruciate ligament, one of the ligaments connecting the femur (thigh bone) and the tibia (shin bone) at the center of the knee joint. The ACL, which limits hyperextension of the knee and prevents the backward sliding of the femur on the tibial plateau, may be injured when the knee is twisted beyond the normal range of motion, e.g., when the knee is twisted while bending and weaving during skiing and other sports activities. ACL injuries may take the form of total or partial tears.

Reconstruction is the most common form of surgery for injuries to the ACL and involves replacing the ACL with a graft of autologous or artificial tendon. An autologous tendon graft may be "harvested" from the patient's patellar ligament, which is part of the common tendon of the quadriceps femoris, connecting the patella to the tibia. An alternative autologous tendon graft may be harvested from the semitendinosus tendon, a long tendon running posteriorly and medially along the thigh, connecting the upper femur to the tibia. Traditionally, patellar grafts are harvested with attached bone plugs that can be securely fixed at the ends of a bone tunnel drilled through the tibia and femur using metallic interference screws, a metal screw and washer, or buttons. Drawbacks associated with the use of the patellar tendon, include difficulties in harvesting the tendon and post-operative complications.

More recent success has been found using one or more strands of the triple-stranded semitendinosus tendon, which can be harvested with minimal post-operative complications. The strands can be used alone or in combination with the gracilis tendon, which anatomically runs parallel along the thigh to the semitendinosus tendon. Although semitendinosus tendons are increasingly used in ACL repair, they are difficult to attach to bone, due in part to the absence of associated bone plugs.

The art suggests several techniques for attaching the semitendinosus tendon to bone in ACL repair. One such technique involves suturing the tendon to a button or staple on the exterior of the bone. Drawbacks associated with this method include stretching or failure of the suture, which may be subjected to tensile forces ranging from 13.6-22.7 kilogram-force (30-50 pounds).

Another technique involves attaching a tendon graft to bone using metallic interference screws. Although such metal screws demonstrate stable fixation and good tensile strength, drawbacks associated with their use include distortion of post-operative radiological studies, metal sensitivity associated with permanently implanted metal screws, or additional operations for metal removal.

Another technique involves attaching a tendon graft to an anchor affixed within a tunnel drilled in the bone. One anchor intended for this use is the Mitek Ligament Anchor available from Mitek Surgical Products, Inc. That anchor includes prongs that lodge into the bone after the anchor has been pulled into position by a suture. Drawbacks associated with this attachment means includes that the anchor must be fabricated from metal to insure sufficient holding strength. Moreover, it must be lodged in the cortical layer near the surface of the femur and therefore necessitates the use of long tendon segments.

An object of this invention is to provide improved surgical apparatus for attaching autologous or artificial tendon grafts to bone.

Another object of this invention is to provide improved apparatus for attachment of autologous or artificial tendon grafts (e.g., for ACL repair) in which the attachment means can be fabricated from polymers or bioabsorbable materials without the use of metals.

A related object of this invention is to provide apparatus for attachment of autologous and artificial tendons that minimize drawbacks associated with pullout of tendon grafts in ACL or other reconstructive orthopedic surgery.

### Summary of the Invention

The above objects are those met by the invention, which provides in one aspect an improved apparatus for attaching autologous or artificial tendon grafts to bone during ligament and other reconstructive surgery, including ACL reconstruction. The apparatus allows anchoring the tendon graft in the cancellous portion of the bone (i.e., close to the anatomical ACL femoral attachment site), without sutures or without metal.

The anchor assembly comprises an insertion element with a stem and an aperture-containing head proximal to the stem. The aperture is sufficiently large to receive autologous tendons grafts such as semitendinosus tendon or artificial tendons. The assembly also comprises a stabilizing element adapted to be embedded in bone. The stabilizing element comprises a sleeve with a cavity, which can be an elongated axial channel, arranged to receive and hold the insertion element stem.

The anchor assembly is designed to anchor autologous and artificial tendon grafts into a stepped tunnel drilled into bone. The stabilization element is embedded in the bone, optimally in the cancellous portion of the femur or tibia, at the point in which the stepped tunnel changes to the smaller diameter bore. Embedding is accomplished by screwing the stabilization element into the smaller diameter bore, which is located at a point close to the anatomical ACL attachment site. Next, the insertion element with its attached, looped tendon is tapped into the stabilizing element, creating a stable compression fit such that the graft is securely held in the bone. The anchor assembly can also be inserted into the tibia or other bone.

The invention could be used in a method for anchoring autologous and artificial tendon graft to bone by drilling a stepped tunnel into the bones forming a joint, for example, into the tibia and femur where they join at the knee. The stabilizing element is embedded in the narrower end of the tunnel in the interior of the bone, while the insertion element is embedded into the stabilizing element such the wide head of the insertion element is located in the wider portion of the tunnel.

The insertion element, with its attached looped tendon, is pulled into the stabilizing element. This is accomplished by providing a small structure, such as an aperture, slot, barb or hook, on the insertion element, preferably, at its distal end. A K-wire equipped with an eyelet can be used to thread a suture through the skin and bone, and then, through the aforementioned structure. In this fashion, the suture can be used to pull the insertion element into the stabilizing element.

In a related aspect, the stem of the insertion element has a larger outer diameter than the inner diameter of the sleeve of the stabilizing element. Therefore, the insertion element is held by compression or interference fit into the stabilizing element embedded in the bone.

The advantages of the apparatus of the instant invention allow the anchor assembly to overcome limitations of the prior art. The two-piece apertured design enables construction of an anchor assembly to attach autologous or artificial tendon grafts securely within bone without the use of either metal or sutures. By analogy, the anchor assembly takes into account the advantageous properties of both screws and impact-driven pins. Screws are know to be strong attachment devices capable of holding in bone. Therefore, the stabilizing element is arranged and constructed so that screw-like threads anchor it to bone. Impact-driven pins (e.g., nails) have the advantage of not requiring twisting on insertion, but tend to shatter bone and/or hold poorly in bone. Therefore, the insertion element with its attached tendon graft is either tapped or pulled into a stabilizing element and held there firmly by compression fit without twisting the tendon graft undesirably.

These and other aspects of the invention are evident in the drawings and in the description that follows.

### Brief Description of the Drawings

A more complete understanding of the invention may be attained by reference to the drawings, in which:
Figure 1a depicts a frontal view of the bones of the knee and a partially tom anterior cruciate ligament (ACL);
Figure 1b depicts a side view of a method for creating a stepped tunnel through the tibia and partially through the femur for insertion of an anchor assembly according to the invention;
Figure 2 depicts a frontal view of a method for affixing a tendon graft into the tunnel of Figure 1b;
Figure 3 depicts a detailed side view of an embedded anchor assembly; and
Figure 4a-d depicts an detailed view of an anchor assembly.
Figures 5a-5c depict a detailed view of the insertion element of an anchor assembly according to an alternate embodiment of the present invention.

### Detailed Description of the Invention

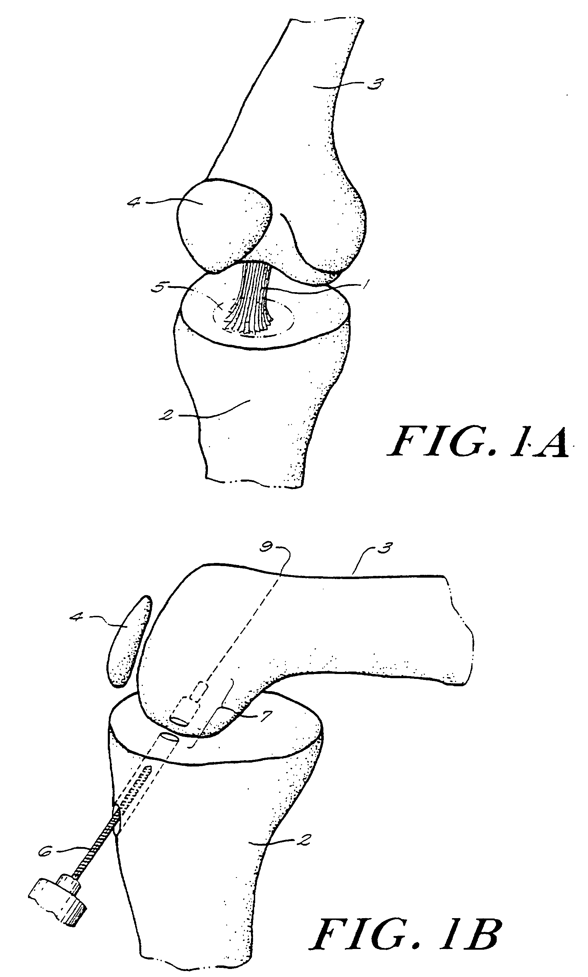

Figure 1a depicts a partially torn Ligament of the knee, e.g., the anterior cruciate ligament (ACL) 1. In the illustration, the ACL is attached to a depression in the anterior intercondylar area (not shown) on the surface of the tibial plateau 5. This tibial attachment lies in front of the anterior intercondylar tubercle and is blended with the anterior extremity of the lateral meniscus (not shown). It passes upward, backward, and laterally to be fixed into the posterior part of the medial surface of the lateral condyle (not shown) of the femur 3. The tibia 2 and the patella 4 are also shown.

Figure 1b depicts a method for creating a stepped tunnel 7 through the tibia 2 and partially through the femur 3 for insertion of an anchor assembly of the invention. In the illustration, a drill 7 is used by the surgeon to drill an tunnel beginning at the anterior surface of the tibia 2 and ending within the cancellous region of the femur 3. The drill tunnel 7 preferably will enter the femur 3 at or near the isometric point (not shown) close to the anatomical ACL attachment site in accordance with the prior art. The angle of the drill tunnel is in accord with that practiced in the prior art for semitendinosus-style ACL repair. The stepped hole is formed by use of a stepped drill bit such that the ledge separating the wider and narrower diameter tunnels lies within the cancellous portion of the femur 3, e.g., within at least 10mm to 70 mm within the femur of the posterior part of the medial surface of the lateral condyle and, preferably, approximately 45 mm of that surface. The drill tunnel 7 may terminate within the cancellous portion of the femur 3, or, in the alternative, the surgeon may elect initially to fully penetrate the femur 3 with a guide wire (not shown), leaving a small exit aperture 9 on the opposing surface of the femur in accordance with the prior art covering ACL reconstructive surgery. It will be appreciated by those skilled in the prior art that the above-described invention is not limited to embedding an anchor assembly in the femur 3 but could also be practiced to embed an anchor in the tibia 2 or in bones comprising other joints, e.g., the ankle or elbow region.

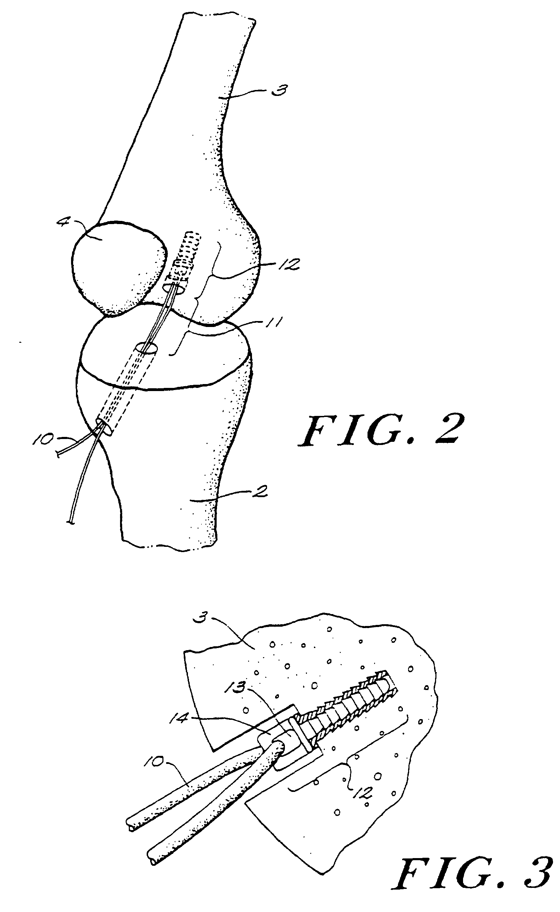

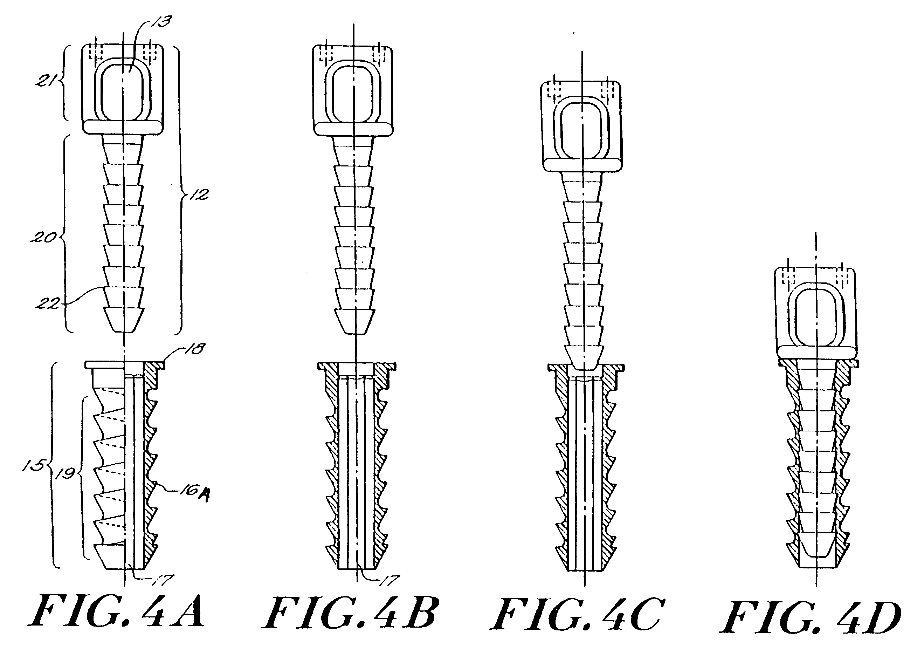

Figure 2 depicts shows a graft anchor assembly 12 of the instant invention embedded in bone, for example in the cancellous layer of the femur 3. A tendon graft 10 is looped through the aperture (see detailed drawing in Figure 3) in a anchor assembly 12 with one or more free ends extending through other bone, for example. through the tibia 2.

Figure 3 depicts in more detail an anchor assembly 12 in operating position embedded in the stepped bone tunnel. The autologous or artificial tendon graft 10 is looped through aperture 13 in the head of the insertion element 14. The stabilizing element 15 is embedded in the bone tunnel, for example by screwing into the stepped tunnel. The insertion element 14 is held in the stabilizing element 15 for example by compression fit.

Figures 4a-d depict an anchor assembly in detail. Figure 4a depicts the stabilizing element 15 which comprises an elongated sleeve 19 containing external protrusions 16A, for example external threads. Stabilizing element 15 has a cavity 17, for example an elongated axial channel 17 extending at least partway from the proximal end of stabilizing element 15. For example, axial channel 17 could extend from the proximal to the distal end of stabilizing element 15. Stabilizing element has a flanged head 18. Stabilizing element 15 is comprised of a biocompatible material, for example implant grade high density polyethylene, low density polyethylene (PE 6010 and PE 2030) and polypropylene (13R9A and 23M2: all made by Rexene, Dallas, Texas) or of a bioabsorbable material, for example poly-1-lactide or such as a lactide-glycolide composition. It may also be comprised of a metal. such as, surgical implant grade steel.

Figure 4a also depicts insertion element 14. Insertion element 14 has an aperture 13 containing head 21 for retaining a ligament replacement. Stem head 21 has an aperture 13 of a size suitable for receiving multiple strands of autologous and/or artificial tendon, but optimally for receiving two or more strands of semitendinosus tendon. The aperture 13 may have dimensions 2.5-8.9 mm (0.10 inches - 0.35 inches) (height) by 1.3-7.6 mm (0.05 - 0.30 inches) (width), and, preferably approximately 5.6 by 4.1 mm (0.220 inches by 0.160 inches). Insertion element 14 has a stem 20, for example an elongated stem 20. The stem has protrusions 22 extending outwardly. Stem protrusions 22 may be inflexible. The diameter of stem 20 is greater than the diameter of axial channel 17 such that stabilizing element 15 is capable of holding the insertion element 14 by compression fit upon insertion of the insertion element 14 into channel 17 of stabilizing element 15. The insertion element 12 can be tapped into the stabilizing element 15 with an emplacement device (not shown). The insertion element 12 is comprised of a biocompatible material, for example implant grade high density polyethylene, low density polyethylene (PE 6010 and PE 2030) and polypropylene (13R9A and 23M2: all made by Rexene, Dallas, Texas) or of a bioabsorbable material, for example poly-1-lactide or such as a lactide-glycolide composition. It may also be comprised of a metal, such as, surgical implant grade steel.

Figure 4b depicts axial channel 17 which has a non-cylindrical cross-section (not shown), optimally a polygon such as a hexagon. Other non-cylindrical cross-sections such as a square or pentagon or even oval configurations are also envisioned. A non-cylindrical cross-section of the axial channel 17 is designed such that a emplacement device (not shown) such as a driver (not shown) with a corresponding non-cylindrical diameter can be inserted into a axial channel and turned such that the external threads 16 of the stabilizing element 15 are screwed into and grip the bone. One such driver is, e.g., an Allen wrench.

Figure 4c depicts insertion of the distal end of an insertion element 12 into the axial channel 17 at the proximal end of a stabilizing element 15. The diameter of elongated stem 20 is slightly greater that the diameter of the non-cylindrical axial channel 17 of the stabilizing element. As a result as depicted in Figure 4d, an elongated stem 20 of the insertion element 12 is held tightly in stabilizing element 15, by compression fit into stabilizing element 15 embedded in a stepped bone hole.

Figure 5a depicts an insertion element 10 that can be pulled into the stabilizing element 15 (Figure 4). As above, the insertion element 10 has an aperture 12 containing a head for retaining a ligament replacement and a stem 14 with outwardly expanding protrusions. The diameter of stem is greater than the diameter of axial channel such that stabilizing element 15 is capable of holding the insertion element by compression fit upon insertion of the insertion element into the channel of the stabilizing element. Additionally, the insertion element 10 contains a structure, e.g., aperture 16, suitable for receiving a suture, a wire or other device that can be used to pull the element 10 into the stabilizing element 15 instead of, or in addition to, its being tapped into that element 15.

The aperture 16 or other such structure can be located at any point on the insertion element 10 but is preferably located at the distal end of the insertion element. Thus, for example, in an embodiment in which the stem of the insertion element is approximately 19.05 mm (0.75 inches) long with a diameter of 4.1 mm (0.16 inches), the aperture is located 1.3-5.1 mm (0.05 - 0.20 inches) from the end of the insertion element and preferably 3.04 mm (0.12 inches) from the distal end.

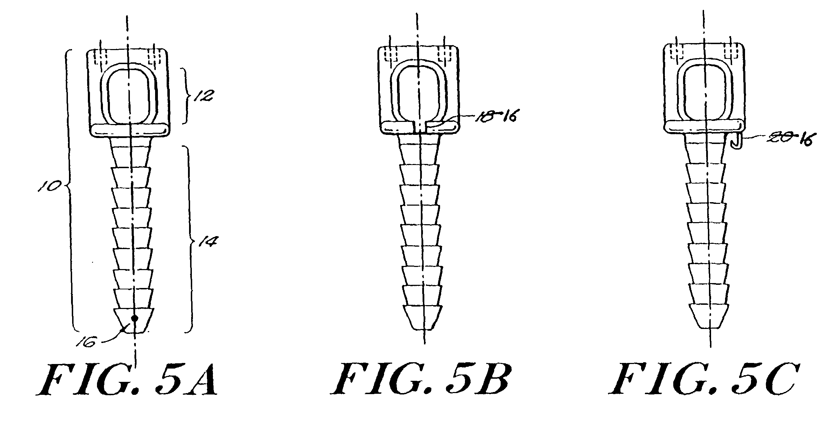

The aperture 16 (or other such structure) is sized sufficiently to accomodate a suture, wire or other pulling device. Those of ordinary skill in the art will of course appreciate that in lieu of an aperture, a slot, barb, hook (as shown in Figures 5b and 5c) or any other structure by which the insertion element can be pulled, can be utilized.

An anchor assembly incorporating an insertion element 10 of Figure 5a is generally implanted as described above. In ACL reconstructive surgery, for example, a tunnel is drilled at the anterior surface of the tibia and ending within the cancellous region of the femur. The drill tunnel preferably enters the femur at or near the isometric point close to the anatomical ACL attachment site in accordance with the prior art. The angle of the drill tunnel is in accord with that practiced in the prior art for semitendinosus-style ACL repair. A stepped hole is formed by use of a stepped drill bit such that the ledge separating the wider and narrower diameter tunnels lies within the cancellous portion of the femur, e.g., within at least 10 mm to 70 mm within the femur of the posterior part of the medical surface of the lateral condyle and, preferably, approximately 45 mm of that surface.

Although the drill tunnel may terminate within the cancellous portion of the femur, a guide wire or K-wire is preferably used to fully penetrate the femur, leaving a small exit aperture on the opposing surface on the femur. The stabilizing element is then embedded in the drilled bone tunnel, for example, by screwing it into the stepped tunnel. At this point, the K-wire (which is preferably equipped with an eyelet at its end) is used to thread a suture through the skin, bone and through the channel of the stabilizing element. The suture is then looped through the aperture, hook, barb, or slot, or other such structure in the insertion element. The insertion element is then pulled into the stabilizing element using that suture. Those skilled in the art will appreciate that a wire, hook or other such apparatus can be used in place of the aforementioned suture.

A further understanding of the preferred practice of the invention may be obtained by reference to the Appendix filed herewith. Those drawings show schematics of a preferred tendon graft anchoring assembly.

Described above are apparatus and methods meeting the objects set forth above. Those skilled in the art will appreciate that the illustrated embodiments are shown and described by way of example only, and that other apparatus incorporation modifications therein fall within the scope of the invention. For example, in addition to ACL reconstruction, the invention can be beneficially applied in connection with other soft tissue-to-bone attachments using bone tunnels, such as (by way of non-limiting example) repair of ligaments and tendons in other joints such as the elbow and ankle. In view of the foregoing, what we claim is:

## Claims

1. An assembly (12) for anchoring soft tissue or artificial grafts (10) in bone, comprising:
an insertion element (14) comprising a stem (20) having an aperture-containing stem head (21) proximal to said stem (20), said stem head aperture (13) being of a size sufficiently large to receive a soft tissue graft (10); and
a stabilizing element (15) adapted to be embedded in bone comprising a sleeve (19), having a cavity (17) arranged and constructed so as to receive and hold said stem (20) by compression fit when the stem (20) is fully inserted into the cavity (17) **characterised in that** said stem has any of an aperture, barb or slot (16) adapted to accept a length of suture by which said insertion element (14) can be pulled into a bone hole.

2. An assembly according to claim 1, wherein said cavity is elongated and has an inner diameter slightly smaller than an outer diameter of said stem.

3. An assembly according to claim 1, wherein at least one of said stem of said insertion element and said sleeve of said stabilization element are elongate and have protrusions on their outer surfaces.

4. An assembly according to claim 3, wherein said protrusions on said stabilizing element comprise threading amenable to being screwed into an opening drilled into bone.

5. An assembly according to claim 1, wherein said sleeve comprises threading amenable to being screwed into an opening drilled into bone.

6. An assembly according to claim 1, wherein said cavity comprises an axial channel, the cross-section of said channel being non-cylindrical, said axial channel extending between proximal and distal ends of said elongate sleeve.

7. An assembly according to claim 1, wherein said stem is an elongated stem and said cavity in said sleeve is an axial channel, said axial channel having a diameter slightly smaller than that of said elongate stem such that said stabilizing element will expand upon insertion of said insertion element into said axial channel.

8. An assembly according to claim 1, wherein a cross-section of said cavity is non-cylindrical, and optionally, wherein said stabilizing element can be deformably expanded to obtain a pressure fit within a bone opening upon insertion of said insertion element into said non-cylindrical aperture of said stabilizing element.

9. The assembly according to claim 1, wherein said insertion and stabilizing elements comprise bio-compatible material.

10. The assembly according to claim 1, wherein said sleeve is an elongate sleeve with external threads and said cavity is an axial channel passing through said elongate sleeve, said axial channel having a non-cylindrical cross-section such that an emplacement device can be inserted therein for screwing said threads of said stabilizing element into said bone.

11. The assembly according to claim 1, wherein said stabilizing element has a fanged or flanged proximal end.

12. The assembly according to any of the preceding claims wherein any of an aperture, slot and barb is disposed at the distal end of said insertion element.

13. The assembly according to claim 1, wherein said cavity has a non-cylindrical cross-section corresponding to the non-cylindrical cross-section of an emplacement device.

14. An assembly according to claim 1, wherein said stabilizing element further comprises:
a flange formed on said stabilizing element at the opening of said cavity, said flange being disposable at least partially outside a bone hole in a configuration whereby it will oppose any further movement of said stabilizing element into said bone hole.

15. An assembly according to claim 1, wherein said cavity comprises an axial channel, the cross-section of said cavity being non-cylindrical, such that an emplacement device can be inserted therein for rotating said stabilizing element about an axis of said axial cavity, said cavity extending between proximal and distal ends of said elongated sleeve.

16. The assembly according to claim 15, wherein said stabilizing element can be deformably expanded to obtain a pressure fit within a bone opening upon insertion of said insertion element into said non-cylindrical aperture of said stabilizing element.

## Patentansprüche

1. Anordnung (12) zum Verankern von Weichteilgewebe oder künstlichen Transplantaten (10) in einem Knochen, umfassend:
ein Einsatzelement (14), das einen Schaft (20) mit einem eine Öffnung enthaltenden Schaftkopf (21), der zu dem Schaft (20) proximal ist, umfasst, wobei die Schaftkopföffnung (13) von einer Größe ist, die ausreichend groß ist, um ein Weichteilimplantat (10) aufzunehmen; und
ein Stabilisierungselement (15), das angepasst ist, um in einem Knochen eingebettet zu werden, umfassend eine Hülse (19) mit einem Hohlraum (17), der angeordnet und konstruiert ist, um den Schaft (20) durch einen Kompressionssitz aufzunehmen und zu halten, wenn der Schaft (20) im Hohlraum (17) voll eingesetzt ist, **dadurch gekennzeichnet, dass** der Schaft eine Öffnung, einen Widerhaken oder einen Schlitz (16) aufweist, wobei die Öffnung, der Widerhaken oder der Schlitz derart ausgebildet ist, um eine Länge von Nahtmaterial aufzunehmen, wodurch das Einsatzelement (14) in ein Knochenloch gezogen werden kann.

2. Anordnung nach Anspruch 1, bei der der Hohlraum langgestreckt ist und einen Innendurchmesser aufweist, der etwas kleiner als ein Außendurchmesser des Schafts ist.

3. Anordnung nach Anspruch 1, bei der mindestens eines von dem Schaft des Einsatzelements und der Hülse des Stabilisierungselements langgestreckt ist und Vorsprünge auf seinen Außenseiten aufweist.

4. Anordnung nach Anspruch 3, bei der die Vorsprünge auf dem Stabilisierungselement einen Gewindeverlauf umfassen, der geeignet ist, um in eine Öffnung eingeschraubt zu werden, die im Knochen gebohrt ist.

5. Anordnung nach Anspruch 1, bei der die Hülse einen Gewindeverlauf umfasst, der geeignet ist, um in eine Öffnung eingeschraubt zu werden, die im Knochen gebohrt ist.

6. Anordnung nach Anspruch 1, bei der der Hohlraum einen axialen Kanal umfasst, wobei der Querschnitt des Kanals nichtzylindrisch ist, wobei sich der axiale Kanal zwischen einem proximalen und distalen Ende der langgestreckten Hülse erstreckt.

7. Anordnung nach Anspruch 1, bei der der Schaft ein langgestreckter Schaft ist und der Hohlraum in der Hülse ein axialer Kanal ist, wobei der axiale Kanal einen Durchmesser aufweist, der etwas kleiner als derjenige des langgestreckten Schafts ist, so dass sich bei Einsetzen des Einsatzelements in den axialen Kanal das Stabilisierungselement expandiert.

8. Anordnung nach Anspruch 1, bei der ein Querschnitt des Hohlraums nichtzylindrisch ist und, fakultativ, bei der das Stabilisierungselement verformbar expandiert werden kann, so dass bei Einsetzen des Einsatzelements in die nichtzylindrische Öffnung des Stabilisierungselements ein Presssitz in einer Knochenöffnung erhalten wird.

9. Anordnung nach Anspruch 1, bei der das Einsatz- und Stabilisierungselement biokompatibles Material umfassen.

10. Anordnung nach Anspruch 1, bei der die Hülse eine langgestreckte Hülse mit Außengewindegängen ist und der Hohlraum ein axialer Kanal ist, der durch die langgestreckte Hülse hindurch verläuft, wobei der axiale Kanal einen nichtzylindrischen Querschnitt aufweist, so dass ein Platzierungsgerät darin eingesetzt werden kann, um die Gewindegänge des Stabilisierungselements in den Knochen zu schrauben.

11. Anordnung nach Anspruch 1, bei der das Stabilisierungselement ein mit Verankerungen oder Flansch versehenes proximales Ende aufweist.

12. Anordnung nach einem der vorangehenden Ansprüche, bei der jegliches von einer Öffnung, einem Schlitz und einem Widerhaken am distalen Ende des Einsatzelements angeordnet ist.

13. Anordnung nach Anspruch 1, bei der der Hohlraum einen nichtzylindrischen Querschnitt entsprechend dem nichtzylindrischen Querschnitt eines Platzierungsgeräts aufweist.

14. Anordnung nach Anspruch 1, bei der das Stabilisierungselement weiter umfasst:
einen Flansch, der auf dem Stabilisierungselement an der Öffnung des Hohlraums gebildet ist, wobei sich der Flansch mindestens teilweise außerhalb eines Knochenlochs in einer Konfiguration anordnen lässt, wodurch er jeglicher weiteren Bewegung des Stabilisierungselements in das Knochenloch Widerstand entgegensetzt.

15. Anordnung nach Anspruch 1, bei der der Hohlraum einen axialen Kanal umfasst, wobei der Querschnitt des Hohlraums nichtzylindrisch ist, so dass ein Platzierungsgerät darin eingesetzt werden kann, um das Stabilisierungselement um eine Achse des axialen Hohlraums zu drehen, wobei sich der Hohlraum zwischen einem proximalen und distalen Ende der langgestreckten Hülse erstreckt.

16. Anordnung nach Anspruch 15, bei der das Stabilisierungselement verformbar expandiert werden kann, so dass bei Einsetzen des Einsatzelements in die nichtzylindrische Öffnung des Stabilisierungselements ein Presssitz in einer Knochenöffnung erhalten wird.

## Revendications

1. Dispositif (12) d'ancrage de greffes (10) artificielles ou de tissu mou dans un os, comprenant :
un élément d'insertion (14) comprenant une tige (20) présentant une tête (21) de tige, dotée d'une ouverture, du coté proximal de ladite tige (20), ladite ouverture (13) de tête de tige ayant une taille suffisante pour recevoir une greffe (10) de tissu mou; et
un élément de stabilisation (15) destiné à être incorporé dans l'os, lequel élément de stabilisation comprend un manchon (19) dans lequel est ménagé une cavité (17) conformée de manière à recevoir et maintenir ladite tige (20) avec ajustage par compression lorsque la tige (20) est totalement insérée dans la cavité (17),
**caractérisé en ce que** ladite tige comporte un élément (16) quelconque parmi une pointe ou une fente ou une ouverture destinée à recevoir une longueur de suture permettant de tirer ledit élément d'insertion (14) dans un trou ménagé dans l'os.

2. Dispositif selon la revendication 1, dans lequel ladite cavité est allongée et a un diamètre intérieur légèrement inférieur au diamètre extérieur de ladite tige.

3. Dispositif selon la revendication 1, dans lequel au moins un élément parmi ladite tige dudit élément d'insertion et ledit manchon dudit élément de stabilisation est allongé et comporte des saillies sur sa surface extérieure.

4. Dispositif selon la revendication 3, dans lequel lesdites saillies sur ledit élément de stabilisation comprend un filetage destiné à être vissé dans une ouverture percée dans l'os.

5. Dispositif selon la revendication 1, dans lequel ledit manchon comprend un filetage destiné être vissé dans une ouverture percée dans l'os.

6. Dispositif selon la revendication 1, dans lequel la cavité comprend un canal axial, la section dudit canal axial n'étant pas cylindrique et ledit canal axial s'étendant entre les extrémités proximale et distale dudit manchon allongé.

7. Dispositif selon la revendication 1, dans lequel ladite tige est une tige allongée et ladite cavité ménagée dans ledit manchon est un canal axial, ledit canal axial ayant un diamètre légèrement inférieur à celui de ladite tige allongée de sorte que ledit élément de stabilisation s'expansera lors de l'insertion dudit élément d'insertion dans ledit canal axial.

8. Dispositif selon la revendication 1, dans lequel la section de ladite cavité n'est pas cylindrique, et en option, dans lequel ledit élément de stabilisation peut être expansé de façon déformable afin d'obtenir un ajustement par pression à l'intérieur de l'ouverture ménagée dans l'os lors de l'insertion dudit élément d'insertion dans ladite ouverture non cylindrique dudit élément de stabilisation.

9. Dispositif selon la revendication 1, dans lequel lesdits éléments d'insertion et de stabilisation sont en matériau biocompatible.

10. Dispositif selon la revendication 1, dans lequel ledit manchon est un manchon allongé comportant des filetages extérieurs et ladite cavité est un canal axial ménagé à travers ledit manchon allongé, ledit canal axial ayant une section non cylindrique de sorte qu'un dispositif de mise en place peut être insérée à l'intérieur pour visser les filets de l'élément de stabilisation dans ledit os.

11. Dispositif selon la revendication 1, dans lequel ledit élément de stabilisation comporte une extrémité proximale à crochet ou à bride.

12. Dispositif selon l'une quelconque des revendications précédentes, dans lequel un élément quelconque parmi une ouverture, une fente et une pointe est placé à l'extrémité distale dudit élément d'insertion.

13. Dispositif selon la revendication 1, dans lequel ladite cavité a une section non cylindrique correspondant à la section non cylindrique d'un dispositif de mise en place.

14. Dispositif selon la revendication 1, dans lequel ledit élément de stabilisation comprend en outre :
une bride formée sur ledit élément de stabilisation au niveau de l'ouverture de ladite cavité, ladite bride étant mise en place au moins partiellement à l'extérieur d'un trou ménagé dans un os dans une configuration permettant de s'opposer à tout mouvement supplémentaire dudit élément de stabilisation dans ledit trou ménagé dans l'os.

15. Dispositif selon la revendication 1, dans lequel ladite cavité comprend un canal axial, la section de ladite cavité étant non cylindrique, de sorte qu'un dispositif de mise en place peut être insérée à l'intérieur pour faire tourner ledit élément de stabilisation autour d'un axe de ladite cavité axiale, ladite cavité s'étendant entre des extrémités proximale et distale dudit manchon allongé.

16. Dispositif selon la revendication 15, dans lequel ledit élément de stabilisation peut être expansé de façon déformable pour obtenir un ajustement par pression à l'intérieur d'une ouverture ménagée dans l'os lors de l'insertion dudit élément d'insertion dans ladite ouverture non cylindrique dudit élément de stabilisation.
